Europäisches Patentamt

European Patent Office

Office européen des brevets

(19)

(11) **EP 0 904 298 B1**

(12) **EUROPÄISCHE PATENTSCHRIFT**

(45) Veröffentlichungstag und Bekanntmachung des Hinweises auf die Patenterteilung:
**03.04.2002 Patentblatt 2002/14**

(51) Int Cl.$^7$: **C08B 31/12**, A61K 9/22

(21) Anmeldenummer: **97926983.4**

(86) Internationale Anmeldenummer:
**PCT/DE97/01138**

(22) Anmeldetag: **06.06.1997**

(87) Internationale Veröffentlichungsnummer:
**WO 97/46592 (11.12.1997 Gazette 1997/53)**

(54) **NAHEZU UNVERNETZTE CARBOXYMETHYLSTÄRKE, VERFAHREN ZU DEREN HERSTELLUNG, VERWENDUNG ALS RETARDIERUNGSMITTEL, SOWIE EIN RETARDIERENDES ARZNEIMITTEL**

SUBSTANTIALLY UNCROSSLINKED CARBOXYMETHYL STARCH, PROCESS FOR THE PREPARATION THEREOF, USE AS A RETARDING AGENT AND A RETARDING DRUG

CARBOXYMETHYL-AMIDON SENSIBLEMENT NON RETICULE, SON PROCEDE DE FABRICATION, SON UTILISATION COMME AGENT DE RETARDEMENT ET MEDICAMENT A EFFET DE RETARD

(84) Benannte Vertragsstaaten:
**AT CH DE ES FR GB IE IT LI NL**

(30) Priorität: **06.06.1996 DE 19622790**

(43) Veröffentlichungstag der Anmeldung:
**31.03.1999 Patentblatt 1999/13**

(73) Patentinhaber: **CHP Carbohydrate Pirna GmbH & Co. Kg**
**01796 Pirna (DE)**

(72) Erfinder: **LOCHNER, Thomas**
**D-01796 Pirna (DE)**

(74) Vertreter: **Uhlemann, Henry, Dipl. Chem. et al Patentanwalt,**
**Würzburger Strasse 51**
**01187 Dresden (DE)**

(56) Entgegenhaltungen:
**EP-A- 0 391 852          US-A- 2 599 620**

- **CHEMICAL ABSTRACTS, vol. 96, no. 26, 1982 Columbus, Ohio, US; abstract no. 219608v, Seite 111; Spalte r; XP002043120 & JP 82 031 901 A (SHIKISHIMA SPINNING CO LTD) 20.Februar 1982**
- **CHEMICAL ABSTRACTS, vol. 105, no. 12, 1986 Columbus, Ohio, US; abstract no. 99458b, Seite 109; Spalte r; XP002043121 & JP 61 043 601 A (DAIICHI KOGYO SEIYAKU) 3.März 1986**
- **CHEMICAL ABSTRACTS, vol. 93, no. 10, 8.September 1980 Columbus, Ohio, US; abstract no. 101448, "studies on tablets. XXX. Carboxymethylstarch in tablets with ketophenylbutazone" XP002043122 & KRALOVA K. ET AL.: FARM. OBZ., Bd. 49, Nr. 4, 1980, Seiten 169-175,**
- **CHEMICAL ABSTRACTS, vol. 106, no. 18, 4.Mai 1987 Columbus, Ohio, US; abstract no. 143918, "Manufacture of aminophylline tablets with sustained release" XP002043123 & LICHNEROVA I. ET AL.: FARM. OBZ., Bd. 56, Nr. 1, 1987, Seiten 11-19,**

**Beschreibung**

Die Erfindung betrifft eine nahezu unvernetzte Carboxymethylstärke, ein Verfahren zu deren Herstellung, die Verwendung dieser modifizierten Carboxymethylstärke als Retardierungsmittel sowie ein retardierendes Arzneimittel.

Carboxymethylstärken mit unterschiedlichem Vernetzungsgrad und unterschiedlicher Viskosität werden seit langem in unterschiedlichen Bereichen der Lebensmittel- und Pharmaindustrie sowie Kosmetikindustrie, Zigarettenindustrie und für spezielle technische Anwendungen eingesetzt. Hergestellt werden Carboxymethylstärken u.a. durch Umsetzung nativer Stärke mit Natronlauge und Monochloressigsäure in Gegenwart von alkoholischen Quellungsinhibitoren. Unter Zusatz von z. Bsp. Epichlorhydrin oder Dichloressigsäure erfolgt dann eine Vernetzung der Carboxymethylstärke. Die Vernetzung dient dabei zur Verbesserung und Stabilisierung der Viskositäts- und Quelleigenschaften der Carboxymethylstärke. Erreicht werden dabei weiße fließfähige, sehr hygroskopische Pulver, die in Wasser quellen. Es entstehen entweder milchig trübe Suspensionen oder Gele. Aufgrund der Quelleigenschaften vernetzter Carboxymethylstärken werden diese Stärken unter anderem in der Pharmazie als Tablettenspreng- oder -zerfallhilfsmittel eingesetzt.

Gerade weil Carboxymethylstärken aufgrund ihrer natürlichen Herkunft und ökologischen Verträglichkeit ein weites Einsatzgebiet haben, existiert weiterhin ein erheblicher Bedarf an solchen Stärkederivaten mit neuen Eigenschaften. Die Aufgabe der Erfindung besteht deshalb in der Herstellung einer neuen Carboxymethylstärke.

Erfindungsgemäß wird die Aufgabe durch eine nahezu unvernetzte Carboxymethylstärke gelöst, die sich durch eine niedrige Viskosität auszeichnet, deren gelartige, nicht milchig trübe, sondern klare Lösung nicht sedimentiert.

Unter nahezu unvernetzter Carboxymethylstärke wird erfindungsgemäß Carboxymethylstärke verstanden, die durch Veretherung nativer Stärke in Gegenwart alkoholischer Quellungsinhibitoren unter Ausschluß von Vernetzungsmitteln erhalten und keiner zusätzlichen Vernetzung unterworfen wird. Diese nahezu unvernetzte Carboxymethylstärke Einfügung auf Seite 1 zwischen 2. und 3. Absatz

[0006] Aus US-A-2,599,620 ist ein Verfahren zur Herstellung von Stärkeethern bekannt, bei dem zunächst Methanol und Chloressigsäure vermischt und erhitzt und dann getrocknete Kartoffelstärke eingerührt wird. Erst anschließend wird Natronlauge zudosiert. Diese Verfahrensweise bewirkt, daß eine Derivatisierung der so verarbeiteten Stärke nur an deren Oberfläche auftritt. In CHEM. ABSTR. Vol. 96, 219608v (1982) ist ein Verfahren zur Herstellung von Carboxymethylstärke auf Basis von Maisstärke mittels einer Festphasenreaktion beschrieben. Die Festphasenreaktion führt zu einem Molekülabbau und einem hohen Substitutionsgrad. Aus CHEM. ABSTR. Vol. 105, 99459c (1986) ist ein Verfahren bekannt, bei dem Isopropanol als Suspensionsmittel verwendet wird. Erhalten wird Carboxymethylstärke mit hohem DS-Wert und kristallinen Eigenschaften. In CHEM. ABSTR. Vol. 93, 101448 (1980) und in CHEM. ABSTR. Vol. 106, 143918 (1987) wird auf Arzneimittel mit verzögerter Wirkstofffreisetzung hingewiesen, bei denen Karbamylopektin, Amylin PM 30 und Ethocel STD 100 als Tablettenbindemittel verwendet werden. Mit Erhöhung der Bindemittelkonzentrationen wurde eine retardierende Wirkung beobachtet.

EP-A-0391 852 offenbart eine pharmazeutische Zusammensetzung für die rektale oder vaginale Applikation von Arzneimitteln, welche mindestens ein Hydrokolloid als Trägersubstanz enthält und welches durch Interaktion mit der örtlichen Körperflüssigkeit einen raschen Zerfall der Applikationsform bewirkt. Die im Hydrokolloid enthaltene Carboxymethylstärke entfaltet dabei keine retardierende Wirkung sondern bewirkt einen raschen Zerfall der Applikationsform durch Interaktion mit der örtlichen Körperflüssigkeit.
bildet in wässrigen Lösungen klare Gele, die nicht sedimentieren, eine niedrige Viskosität und geringe Empfindlichkeit gegenüber Scherbeanspruchung aufweisen. Weiterhin sind wässrige Lösungen der erfindungsgemäßen Carboxymethylstärke in ihrer Viskosität frei einstellbar.

Die erfindungsgemäße, nahezu unvernetzte Carboxymethylstärke wird so hergestellt, daß native, unvernetzte Stärke in nahezu wasserfreiem alkoholischem Lösemittel suspendiert und mit NaOH alkalisiert und anschließend mittels Monochloressigsäure bei einer Temperatur von 30 bis 40 °C verethert wird. Das so erhaltene Rohprodukt wird mit einer Säure, vorzugsweise Essigsäure oder Salzsäure, neutralisiert, durch Waschen mit wässrigem alkoholischen Lösungsmittel entsalzt, anschließend mehrfach mit nahezu wasserfreiem alkoholischen Lösungsmittel gewaschen und bei Temperaturen unter 80 °C, vorzugsweise bei 60-80 °C etwa 2 bis 3h schonend unter Vakuum getrocknet.

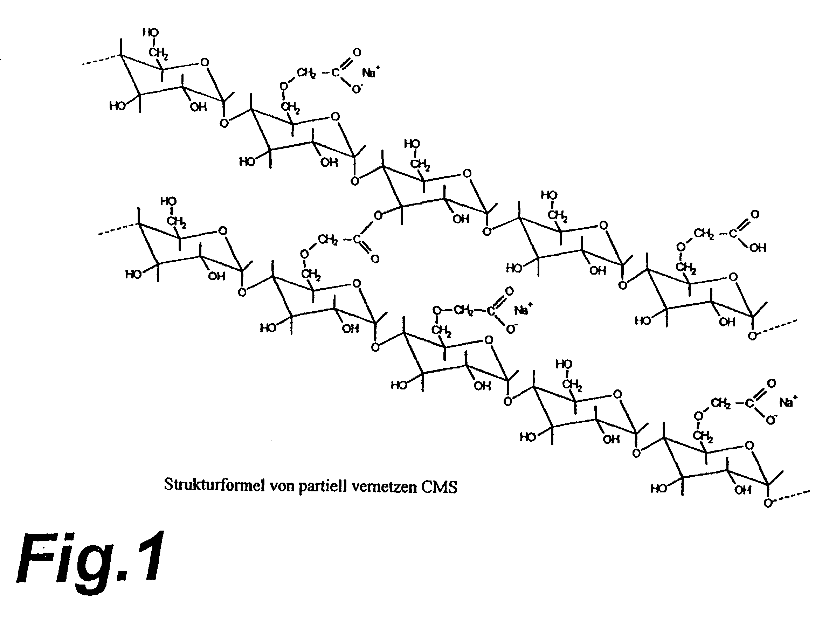

Die Reaktion wird unter Ausschluß von chemischen Vernetzungsmitteln bei Temperaturen unter 80 °C in allen Verfahrensstufen so geführt, daß auch eine physikalische Vernetzung der eingesetzten Stärke und des veretherten Stärkederivates nahezu ausgeschlossen wird. Die sich an die Veretherung anschließende Neutralisation bewirkt eine Umwandlung der Carboxymethylsubstituenten von der Na-Form in die H-Form. Über die H-Form ist eine intermolekularen Vernetzung möglich, wobei nach dem erfindungsgemäßen Verfahren nur eine sehr geringe Vernetzung erfolgt. Figur 1 zeigt eine solche intermolekulare Vernetzung.

Die erfindungsgemäße Carboxymethylstärke ist aufgrund ihrer neuen Eigenschaften vielfältig einsetzbar. Überraschend ist, daß die erfindungsgemäße, nahezu unvemetzte Carboxymethylstärke als Hilfsstoff zur Retardierung in Arzneimitteln geeignet ist. Bislang wurden modifizierte Carboxymethylstärken aufgrund ihrer Quellfähigkeit als Tablettensprengmittel eingesetzt.

Erfindungsgemäß läßt sich eine Arzneimittelzubereitung mit verzögerter Wirkstofffreisetzung so formulieren, daß sie neben dem pharmakologischen Wirkstoff und üblichen Hilfsstoffen, wie Füllstoff, Fließmittel, Gleitmittel usw. 5 bis 25 Ma% nahezu unvernetzter Carboxymethylstärke enthält.

Anhand nachfolgender Ausführungsbeispiele und Darstellungen soll die Erfindung näher erläutert werden. Im einzelnen bezeichnen

    Fig. 1 Strukturformel
    Fig. 2 REM-Aufnahme
    Fig. 3 FTIR-Spektrum
    Fig 4 Viskositätsmeßdaten
    Fig 5 Viskositätsdiagramme Produkt nach Ausführungsbeispiel 1
    Fig 6 Viskositätsdiagramme UAP 5000
    Fig 7 Diagramm Wirkstofffreisetzung Diclofec-Na
    Fig. 8 Diagramm Wirkstofffreisetzung bei Trocken- und Feuchtgranulierung
    Fig. 9 Diagramm Wirkstofffreisetzung Verapamil
    Fig. 10 Diagramm Wirkstofffreisetzung Theophyllin

**Ausführungsbeispiel 1**

In einem Rührbehälter werden in 1500 ml wasserfreies Methanol, 850 g Kartoffelstärke mit 450 ml 45%iger Natronlauge 2 h alkalisiert und anschließend unter Zusatz 300 g Monochloressigsäure bei einer Temperatur von 35 bis 40°C umgesetzt. Nach einer Verweilzeit von 10 bis 15 Stunden wird die erhaltene Suspension gewaschen, mit Essigsäure neutralisiert, durch Waschen mit wässrigem Methanol entsalzt und mit wasserfreiem Methanol mehrfach nachgewaschen. Der Feststoff wird abgetrennt und bei einer Temperatur von 70 bis 80 °C in einem Vakuumschaufeltrockner 3h getrocknet und anschließend gekühlt.

Nach diesem Verfahren wird ein Produkt mit folgenden Charakteristika erhalten:

Aussehen: feines, nahezu weißes freifließendes Pulver

    pH-Wert: 5,5 - 7,5
    Molekulargewicht: $1,1558 \cdot 10^8$ g/mol
    Natriumchlorid (%): < 1
    Natriumglykolat (%): < 2,0
    Na-Gehalt (im Stärkeglykolat): (%) 4,0 - 4,5
    Trockungsverlust (%): <10%
    DS-Wert 0,15 - 0,3
    Gelbildung: bildet in 2%iger wässeriger Lösung klare, niedrigviskose Gele
    Viskosität: gemessen mit dem Kegel-Platte System (4 cm Durchmesser, 2° Kegel, 54 µm Spalt, T= 25°C, Schergeschwindigkeit 50/s) = 630 mPa·s

Das nach Ausführungsbeispiel 1 hergestellte Produkt wurde mittels Rasterelektronenmikroskop und FTIR-Spektroskop Fa. Bruker, Vector 22 untersucht. Gemessen und ausgewertet wurde auch das Viskositätsverhalten des nach Ausführbeipiel 1 hergestellten Produktes, hier mit UAP 100 bezeichnet, in 2%-iger Lösung. Dabei wurde mit dem Viskositätsverhalten eines handelsüblichen chemisch und physikalisch modifizierten Stärkederivates UAP-5000 der Fa. Chemische Fabrik Pirna Copitz GmbH verglichen.

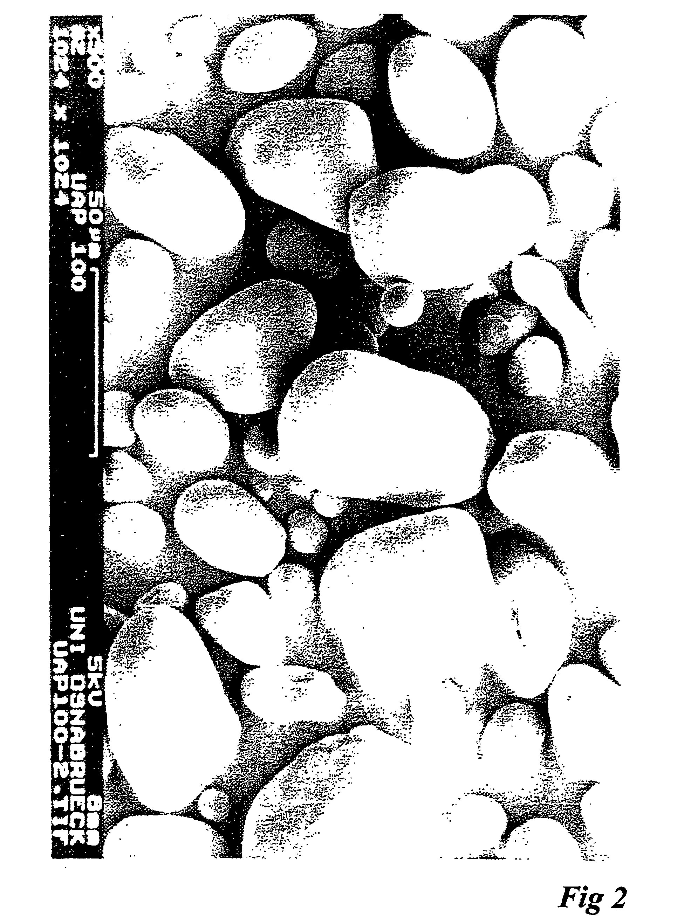

Die als **Fig. 2** beigefügte REM-Aufnahme zeigt ein mit nativer Kartoffelstärke übereinstimmendes Erscheinungsbild. Die für Kartoffelstärke typische Komform ist erhalten geblieben.

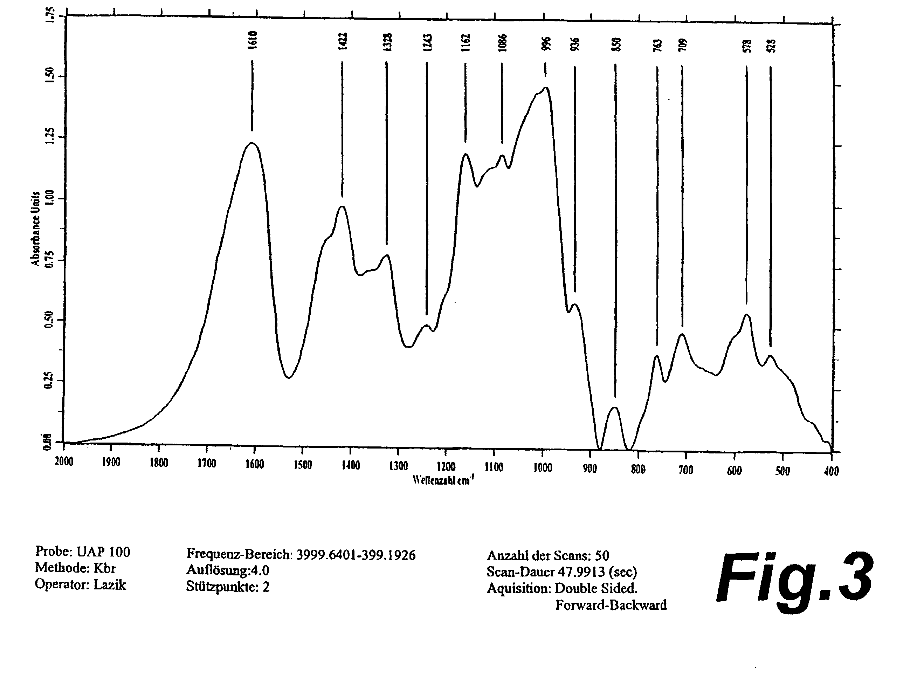

Das als **Fig. 3** beigefügte FTIR-Spektrum korrespondiert mit den IR-Spektren bekannter Carboxymethylstärken. Dennoch unterscheidet sich die erfindungsgemäße Carboxymethylstärke.

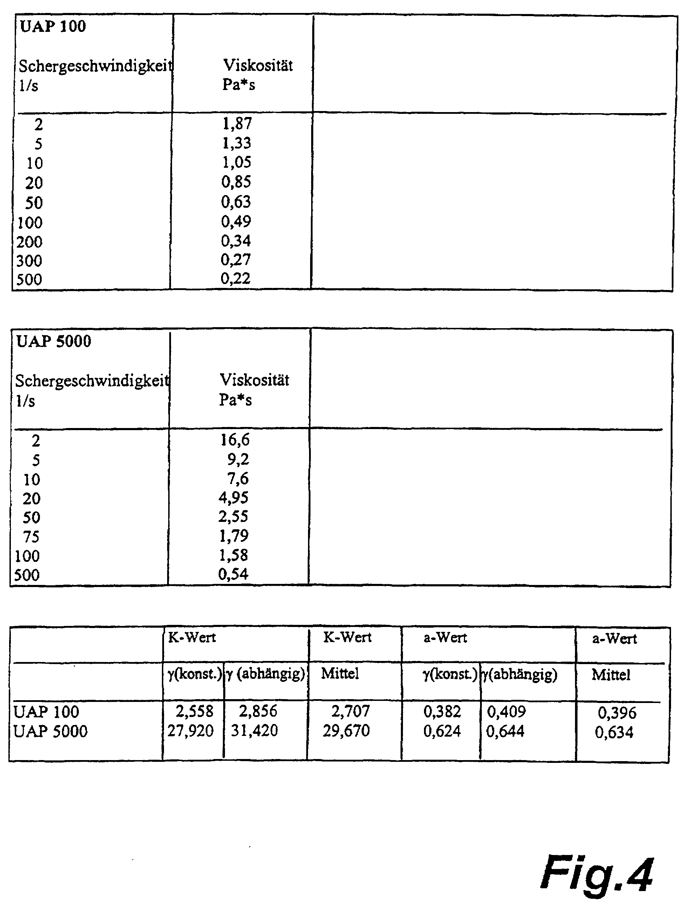

Aus den in **Fig. 4** aufgeführten Viskositätsdaten kann eine wesentlich verringerte Ausgangsviskosität des nach Ausführungsbeispiel 1 hergestellten Produktes gegenüber dem Produkt UAP 5000 entnommen werden. Der ebenfalls geringere a-Wert ist ein Maß für die verringerte Empfindlichkeit des nach Ausführungsbeispiel 1 hergestellten Produktes gegenüber einer Scherkraftbeanspruchung.

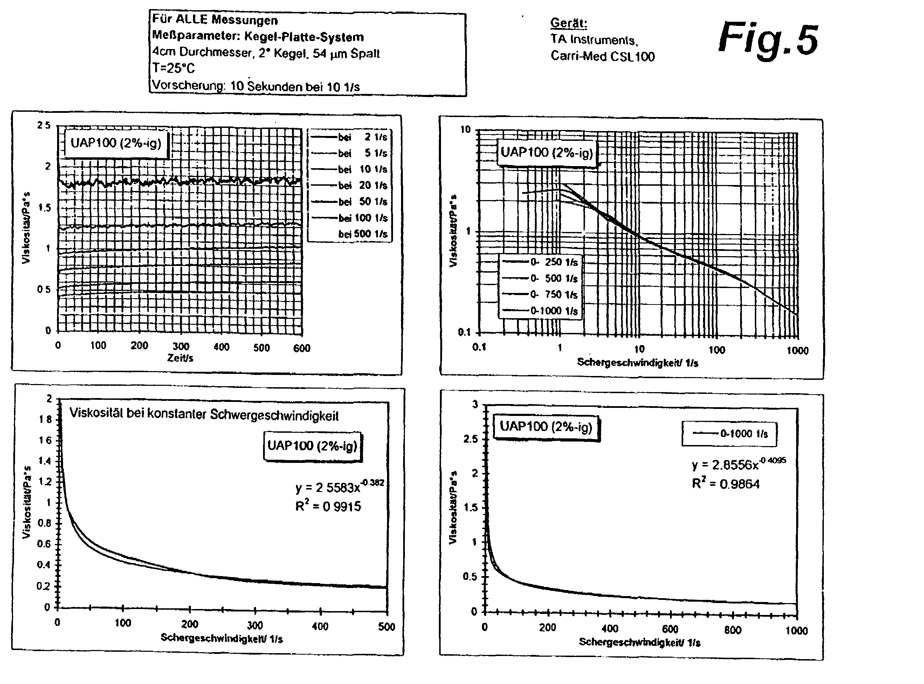

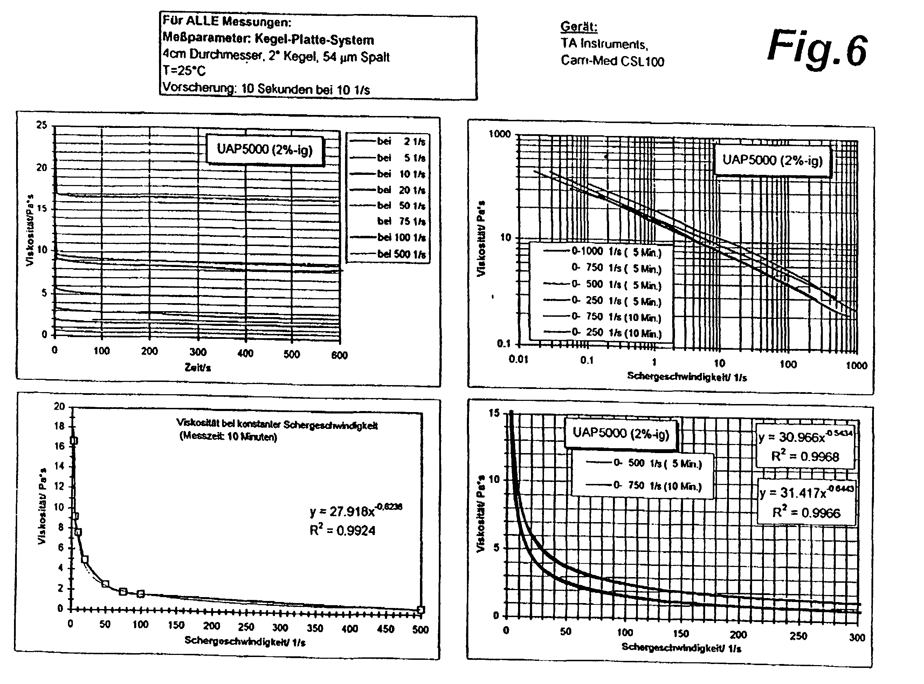

**Fig. 5 und 6** beinhalten Viskositätsdiagramme von UAP 5000 und des nach Ausführungsbeispiel 1 hergestellten Produktes, hier mit UAP 100 bezeichnet. Im Diagramm jeweils links oben ist die Viskosität gegen die Zeit bei konstanter Schergeschwindigkeit auftragen. Im Diagramm jeweils rechts oben sind die Viskositätsdaten und die Schergeschwindigkeit logarithmisch dargestellt (Schergeschwindigkeitsverlauf). Die auf den Fig. 5-6 unten aufgeführten Diagramme zeigen zum einen die Funktion der Viskosität bei konstanter Schergeschwindigkeit und zum anderen die Funktion bei variabler Schergeschwindigkeit. Die Darstellungen beruhen auf Meßdaten, die mit dem Kegel-Platte System und den Meßbedingungen 4 cm Durchmesser, 2° Kegel, 54 um Spalt, T= 25°C, Vorscherung 10 s bei 10 l/s aufgenommen wurden. Als Meßgerät wurde ein Meßgerät Carri Med CSL 100 der Fa. TA-Instruments verwendet.

Um Viskositätsverhalten zu charakterisieren, arbeitet man nach zwei verschiedenen Verfahren. Zum einen wird bei konstanter Schergeschwindigkeit die Viskosität beobachtet (Fig. 5-6, Diagramme jeweils oben links). Im Normalfall stellt sich bei konstanter Schergeschwindigkeit nach kurzer Zeit eine bestimmte Viskosität ein, die sich dann nicht ändert. Zum anderen gibt es die Möglichkeit, die Viskosität bei zunehmender Schergeschwindigkeit zu beobachten, wobei die Zeit hierbei keine Rolle spielt, denn bei Geschwindigkeitsverläufen von 0...250 l/s wird die Messung schneller beendet als bei 0.....1000 1/s.

Im Normalfall erkennt man am Schergeschwindigkeitsverlauf die Viskosität des Gels in Abhängigkeit von der Geschwindigkeit sehr gut. Dies ist auch ein übliches Verfahren, um die Empfindlichkeit gegenüber Scherbeanspruchung zu erkennen.

Zur Quantifizierung des Viskositätsverhaltens wird in der Rheologie eine aus verschiedenen Modellen stammende Formel

$$\eta = K \cdot \gamma^{-a}$$

benutzt. Hierbei bezeichnet K die Quasiviskosität bei keiner Scherbeanspruchung. a gibt die Empfindlichkeit gegenüber Scherbeanspruchung wieder. Dabei gilt:

- je größer der a-Wert, desto größer ist die Abhängigkeit von der Schergeschwindigkeit und
- je größer der K-Wert, desto viskoser ist das Verhalten des Gels (unabhängig von der Schergeschwindigkeit).

Durch eine mathematische Umformung in die logarithmische Form erkennt man, daß der a-Wert die Steigung der Kurven in den oberen rechten Abbildungen der Fig. 5 bis 6 darstellt (log $\eta$ = log K - a log $\gamma$). Mit Hilfe der logarithmischen Regression oder der exponentiellen Regression erhält man die K- und a-Werte. Diese sind in den unteren Abbildungen der Fig. 5 bis 6 mit der allgemeinen Formel y = K · x$^{-a}$ dargestellt, wobei y = $\eta$ und x = $\gamma$ gesetzt ist. Der Regressionskoeffizient R gilt dabei als Maß für die Genauigkeit der Regression.

Wie aus Figur 5 und 6 ersichtlich, unterscheiden sich die Produkte UAP 100 und UAP 5000 nicht nur in ihrem jeweiligen Viskositätsverhalten in Abhängigkeit der Schergeschwindigkeit, sondern auch in ihren Schergeschwindigkeitsverläufen. Es ist deutlich zu erkennen, daß UAP 5000 ein wesentlich viskoseres Verhalten aufweist, jedoch gegenüber Scherbeanspruchung empfindlicher reagiert, was aus der Steilheit in der Abbildung rechts oben (Fig. 6) zu entnehmen ist. Dies drückt sich auch in den entsprechenden K- und a-Werten aus, die aus den unteren beiden Abbildungen entnommen werden können. Ersichtlich ist, daß der K-Wert beim Produkt UAP 5000 ca. 10x höher liegt als beim Produkt UAP 100. Hingegen beträgt der a-Wert von UAP 100 nur 60 % des a-Wertes von UAP 5000. Dies bedeutet eine verringerte Empfindlichkeit gegenüber Scherbeanspruchung. Desweiteren läßt sich die Viskosität des erfindungsgemäßen Produktes in Lösung auch frei einstellen.

**Ausführungsbeispiel 2**

Nach Ausführungsbeispiel 1 hergestellte, nahezu unvernetzte Carboxymethylstärke wird mit dem Arzneimittelwirkstoff Diclofenac-Na, Magnesiumstearat als Gleitmittel, hochdispersem $SiO_2$ (Aerosil) als Fließmittel und mikrokristalliner Zellulose (Microcel 101 SP) als Füllmittel in folgender Zusammensetzung gemischt, wobei die Anteile an Carboxymethylstärke (CMS) und microkristalliner Zellulose variert werden:

| | |
|---|---|
| Diclofenac-Na | 33,3 Ma% (100 mg) |
| Mg-Stearat | 2,0 Ma% |
| Aerosil 200 | 1,5 Ma% |

|  | A | B | C | D |
|---|---|---|---|---|
| MICROCEL 101-SP | 63,7 Ma% | 58,7 Ma% | 53,7 Ma% | 43,7 Ma% |
| CMS | - | 5,0 Ma% | 10,0 Ma% | 20,0 Ma% |

Auf dem Wege der Direkttablettierung werden Tabletten in folgender Weise hergestellt:

Die Substanzen werden durch ein Sieb mit einer Maschenweite von 0,5 mm dispergiert, vermischt und auf einer Exzenter-Tablettiermaschine (Korsch EK 0) zu Tabletten (biplan mit Facettenrand, 10 mm Durchmesser) verpreßt. Bei einem Gewicht von 300 mg/Tablette beträgt der Wirkstoffgehalt 100 mg.

Die so hergestellten Tabletten werden nach folgenden Methoden untersucht:

Gewichtsbestimmung

Jeweils 30 Tabletten werden auf einer Analysenwaage (Sauter) gewogen und das mittlere Gewicht berechnet.

Bestimmung der Tablettenhärte

Jeweils 10 Tabletten werden mit einem Härtetestgerät (Heberlein) mit der Methode der Krafteinwirkung (auf die Waagerechte zwischen zwei zusammendrückenden Backen liegende Tablette) auf ihre Druckfestigkeit geprüft. Aus jeweils 10 Einzelwerten wird der Mittelwert berechnet.

Bestimmung der Abriebfestigkeit

Jeweils 20 Tabletten werden auf einer Analysenwaage (Sauter) gewogen und in eine Friabilator-Trommel (Erweka) mit treppenartigen Einbauten gegeben und rotieren 10 min bei 20 UpM. Anschließend wird das Gewicht der Tabletten ohne Staub- und Bruchstückanteile bestimmt. Das Maß des Abriebes wird in %Gewicht von der Tablette ausgedrückt.

Frei setzungsprüfung

Die Wirkstofffreisetzung von Diclofenac-Na 100 mg-Tabletten wird nach der USP-Paddle-Methode in 900 ml Phosphatpuffer bei pH 7,2 und 50 UpM während 8 h geprüft. Der Gehalt an freigesetztem Diclofenac-Na wird photometrisch gemessen.

Die nach Ausführungsbeispiel 2 hergestellten Tabletten weisen folgende Daten auf:

|  | Anteil (%) CMS | Gewicht (mg) | Härte (kp) | Abrieb (%) |
|---|---|---|---|---|
| (A) | 0 | 299 | 10,8 | 0,23 |
| (B) | 5 | 303 | 10,5 | 0,28 |
| (C) | 10 | 303 | 10,6 | 0,28 |
| (D) | 20 | 299 | 11,0 | 0,17 |

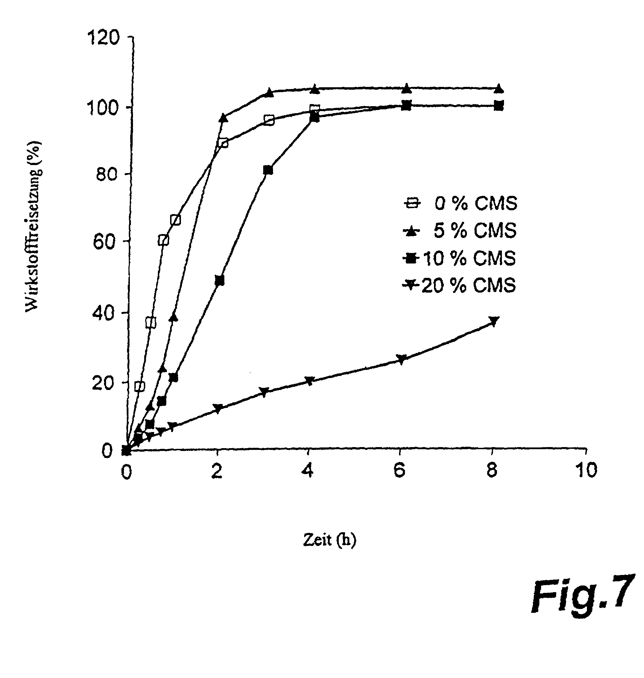

In **Figur 7** sind die bei der Freisetzungsprüfung erreichten Daten aufgeführt. Ersichtlich ist, daß die Tablettenformulierungen mit der erfindungsgemäßen Carboxymethylstärke den Wirkstoff Diclofenac-Na über einen Zeitraum von 8 Stunden verzögert freisetzt im Vergleich zu Tablettenformulierungen ohne die erfindungsgemäße Carboxymethylstärke.

**Ausführungsbeispiel 3**

Unter Verwendung von nach Ausführungsbeispiel 1 hergestellter Carboxymethylstärke (CMS) werden Tabletten nach der Methode der Feuchtgranulierung und der Trockengranulierung hergestellt.

Feuchtgranulierung

Nach Ausführungsbeispiel 1 erhaltene unvernetzte CMS, Diclofenac-Na, mikrokristalline Zellulose (Tabulose 102) werden nach folgender Rezeptur gemischt

| CMS | 15,0 Ma% |
|---|---|
| Diclofenac Na | 33,3 Ma% |
| Tabulose 102 | 48,2 Ma% |

und mit 45 Ma% Wasser (bezogen auf die pulvrigen Substanzen) in einem Mischer (High shear) geknetet und über einen oszillierenden Sternrotor mit Vierkantsieb (1,6 mm) granuliert. Die Granulate werden 2h bei 60 °C getrocknet und über ein Sieb (1,25 mm) abgesiebt (Granulat Feuchtigkeitsgehalt 4%). Das so erhaltene Granulat wird mit 2,0 Ma% Magnesiumstearat und 2,5 Ma% Aerosil gemischt. Analog Ausführungsbeispiel 2 werden Tabletten gepreßt. Dabei werden Tabletten mit einer Härte von 9,1 kp und einer Abriebfestigkeit von 0,8% erreicht (Bestimmung anhand der unter Ausführungsbeispiel 2 dargestellten Methoden).

Trockengranulierung

Die Substanzen werden gemischt, gesiebt und über einen 2-Walzenkompaktor kompaktiert und über eine Siebmaschine (Sieb 1,25 mm) mit oszillierenden Sternrotor zerkleinert (granuliert). Das Granulat wird auf einer Excentertablettiermaschine (Korsch EK 0) zu Tabletten (biplan mit einem Facettenrand 10 mm Durchmesser) verpreßt. Dabei werden Tabletten mit einem Gewicht von 300 mg, Härte 9,1 kp, Abriebfestigkeit 0,6 % erreicht (Bestimmung anhand der unter Ausführungsbeispiel 2 dargestellten Methoden).

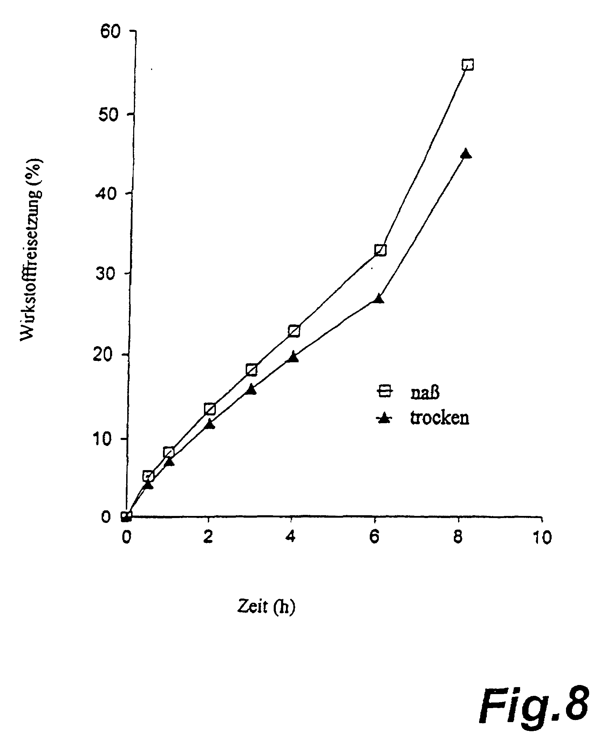

In **Fig. 8** sind die Wirkstofffreisetzungen von Diclofenac-Na nach der USP-Paddle- Methode (50 Upm, 900 ml Phosphatpuffer, pH 7,2) dargestellt. Dabei ist ersichtlich, daß der Wirkstoff Diclofenac-Na über einen Zeitraum von 8 Stunden verzögert freigesetzt wird.

**Ausführungsbeispiel 4**

Analog Ausführungsbeispiel 2 werden Tabletten mit dem pharmakologischen Wirkstoff Verapamil in folgender Zusammensetzung zubereitet:

| Verapamil HCl | 59,1 Ma% |
|---|---|
| CMS | 24,7 Ma% |
| Tabulose 102 | 14,8 Ma% |
| Mg stearat | 0,7 Ma% |
| Aerosil 200 | 0,7 Ma% |

Die Tabletten werden analog der Ausführungsbeispiele 2 und 3 einmal auf dem Weg der Direktablettierung und auf dem Weg der Trockengranulierung hergestellt. Die hergestellten Tabletten enthalten 240 mg Verapamil pro Tablette und weisen entsprechend der bei Ausführungsbeispiel 2 angegebenen Bestimmungsmethoden folgende Daten auf:

| | Direktablettierung | Trockengranulierung |
|---|---|---|
| Tablettengewicht (mg) | 404 | 404 |
| Tablettenhärte (kp) | 6,8 | 11,0 |
| Abriebfestigkeit (%) | 0,7 | 0,2 |

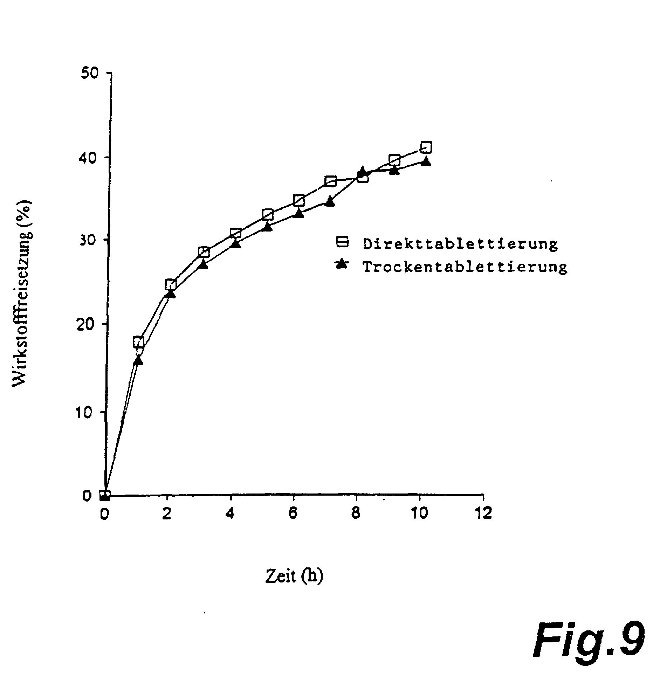

In **Figur 9** sind nach der USP-Paddle-Methode (100 Upm, 900 ml, pH 1,2, 0...2h anschließend pH 6,8) die Wirkstofffreisetzungen graphisch dargestellt. Auch hier zeigt sich, daß der Wirkstoff Veraparnil-HCl über eine Zeit von 8 Stunden verzögert freigesetzt wird.

**Ausführungsbeispiel 5**

Auf dem Wege der Direkttablettierung analog Ausführungsbeipiel 2 wird eine Tablette unter Verwendung der nach Ausführungsbeipiel 1 erhaltenen Carboxymethylstärke (CMS) und des Wirkstoffes Theophyllin mit folgender Zusammensetzung zubereitet:

| Theophyllin | 62,5 Ma% |
|---|---|

(fortgesetzt)

| CMS | 18,8 Ma% |
|---|---|
| Tabulose 102 | 16,7 Ma% |
| Mg-Stearat | 1,0 Ma% |
| Aerosil 200 | 1,0 Ma% |

Die Tabletten enthalten 200 mg Theophyllin pro Tablette und weisen folgende Daten auf:

| Gewicht (mg) | 320 |
|---|---|
| Tablettenhärte (kp) | 9,5 |
| Abrieb (%) | 0,8 |

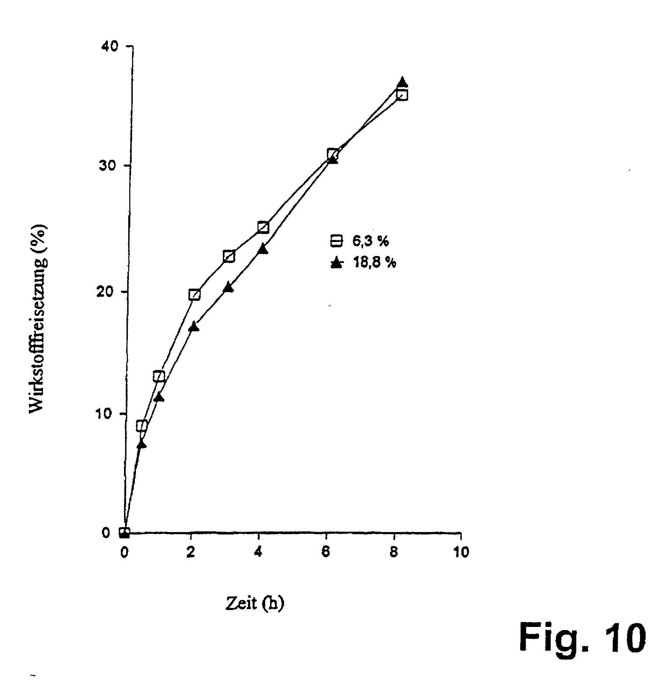

In **Figur 10** ist wiederum die Wirkstofffreisetzung gegenüber der Zeit, ermittelt nach der USP-Paddle-Methode (50 Upm, 900 ml, pH 1,2, 0...2h anschließend pH 6,8), graphisch dargestellt. Hier zeigt sich, daß der Wirkstoff Theophyllin über eine Zeit von 8 Stunden verzögert freigesetzt wird.

**Patentansprüche**

1.  Neue Carboxymethylstärke erhältlich durch Veretherung nativer Stärke in Gegenwart alkoholischer Quellungsinhibitoren unter Ausschluß von Vernetzungsmitteln, **dadurch gekennzeichnet, daß** sie in 2%iger wässriger Lösung eine klare gelartige Lösung bildet, die nicht sedimentiert.

2.  Verfahren zur Herstellung von Carboxymethylstärke nach Anspruch 1, wobei
    native, unvernetzte Stärke in nahezu wasserfreiem alkoholischem Lösemittel suspendiert und mit NaOH alkalisiert und anschließend mittels Monochloressigsäure verethert wird
    **dadurch gekennzeichnet, daß** das erhaltene Rohprodukt gewaschen, mit einer Säure neutralisiert, durch Waschen mit wässrigem alkoholischen Lösungsmittel entsalzt, anschließend mehrfach mit nahezu wasserfreiem alkoholischen Lösungsmittel gewaschen und bei Temperaturen unter 80 °C getrocknet wird und die Reaktion in allen Verfahrensstufen unter Ausschluß von chemischen Vernetzungsmitteln bei Temperaturen unter 80°C geführt wird.

3.  Verwendung von Carboxymethylstärke nach Anspruch 1 als Retardierungsmittel.

4.  Retardierendes Arzneimittel bestehend aus Wirkstoff und Hilfsstoffen, **dadurch gekennzeichnet, daß** es 5 - 25 Ma% Carboxymethylstärke nach Anspruch 1 enthält.

**Claims**

1.  New carboxymethylstarch obtainable by etherification of native starch in the presence of alcoholic swelling inhibitors to the exclusion of cross-linking agents **characterized in that** it forms in a 2-% aqueous solution a clear gel-like solution that does not sediment.

2.  Process for the production of carboxymethylstarch to Claim 1 whereby native, uncrosslinked starch is suspended in almost anhydrous alcoholic solvent and is alkalized with NaOH and subsequently etherified with monochloroacetic acid **characterized in that** the obtained crude product is washed, neutralized with an acid, made free from salts using an aqueous alcoholic solvent, then repeatedly washed with almost anhydrous alcoholic solvent and dried at temperatures below 80 °C and the reaction is conducted at all process stages to the exclusion of chemical cross-linking agents at temperatures below 80 °C.

3.  Use of carboxymethylstarch to Claim 1 as a retarding agent.

4.  Retarding pharmaceutical consisting of active substances and adjuvants **characterized in that** it contains 5-25 wt.-% carboxymethylstarch to Claim 1.

**Revendications**

1. Nouveau carboxyméthylamidon pouvant être obtenu par éthérification d'amidon natif en présence d'inhibiteurs de gonflement alcooliques à l'exclusion d'agents de réticulation, **caractérisé en ce qu'**il forme dans une solution aqueuse à 2 % une solution claire de type gel qui ne sédimente pas.

2. Procédé de préparation de carboxyméthylamidon selon la revendication 1, dans lequel
de l'amidon natif non réticulé est mis en suspension dans un solvant alcoolique presque anhydre, et alcalinisé avec NaOH, puis éthérifié au moyen d'acide monochloroacétique,
**caractérisé en ce que** le produit brut obtenu est lavé, neutralisé avec un acide, dessalé par lavage avec un solvant alcoolique aqueux, puis lavé plusieurs fois avec un solvant alcoolique presque anhydre et séché à des températures inférieures à 80 °C, et **en ce que** la réaction, dans toutes les étapes du procédé, est conduite à l'exclusion de tout agent de réticulation chimique à des températures inférieures à 80 °C.

3. Utilisation de carboxyméthylamidon selon la revendication 1 en tant qu'agent de retardement.

4. Médicament retard composé d'un principe actif et d'additifs, **caractérisé en ce qu'**il contient 5 à 25 % en masse de carboxyméthylamidon selon la revendication 1.

Strukturformel von partiell vernetzen CMS

Fig. 1

*Fig 2*

Fig. 3

Probe: UAP 100
Methode: Kbr
Operator: Lazik

Frequenz-Bereich: 3999.6401-399.1926
Auflösung:4.0
Stützpunkte: 2

Anzahl der Scans: 50
Scan-Dauer 47.9913 (sec)
Aquisition: Double Sided.
Forward-Backward

| UAP 100 | | |
|---|---|---|
| Schergeschwindigkeit 1/s | Viskosität Pa*s | |
| 2 | 1,87 | |
| 5 | 1,33 | |
| 10 | 1,05 | |
| 20 | 0,85 | |
| 50 | 0,63 | |
| 100 | 0,49 | |
| 200 | 0,34 | |
| 300 | 0,27 | |
| 500 | 0,22 | |

| UAP 5000 | | |
|---|---|---|
| Schergeschwindigkeit 1/s | Viskosität Pa*s | |
| 2 | 16,6 | |
| 5 | 9,2 | |
| 10 | 7,6 | |
| 20 | 4,95 | |
| 50 | 2,55 | |
| 75 | 1,79 | |
| 100 | 1,58 | |
| 500 | 0,54 | |

| | K-Wert | | K-Wert | a-Wert | | a-Wert |
|---|---|---|---|---|---|---|
| | $\gamma$(konst.) | $\gamma$ (abhängig) | Mittel | $\gamma$(konst.) | $\gamma$(abhängig) | Mittel |
| UAP 100 | 2,558 | 2,856 | 2,707 | 0,382 | 0,409 | 0,396 |
| UAP 5000 | 27,920 | 31,420 | 29,670 | 0,624 | 0,644 | 0,634 |

*Fig.4*

Fig.5

Für ALLE Messungen
Meßparameter: Kegel-Platte-System
4cm Durchmesser, 2° Kegel, 54 µm Spalt
T=25°C
Vorscherung: 10 Sekunden bei 10 1/s

Gerät:
TA Instruments,
Carri-Med CSL100

EP 0 904 298 B1

Fig.6

Fig.7

Fig.8

**Fig.9**

Fig. 10